# EUROPEAN PATENT APPLICATION

(11) **EP 2 687 264 A1**
(43) Date of publication of application: **22.01.2014**
(21) Application number: 12176723.0
(22) Date of filing: 17.07.2012
(51) Int. Cl.: A61P 15/00, A61K 38/19, A61K 31/525, G01N 33/50

(54) **Macrophage Activating Factor for treatment of endometriosis**

(71) Applicant: Protea Biopharma N.V., 1120 Neder-Over-Heembeek (BE)
(72) Inventor: Roelant, Christiaan, 3000 Leuven (BE); De Meirleir, Kenny, 2800 Mechelen (BE)
(74) Representative: Paemen, Liesbet R.J.

(57) **Abstract**

The present invention relates to methods and compounds for the treatment and prevention of endometriosis and/or symptoms associated with endometriosis in a patient. More particularly, compositions comprising a Macrophage Activating Factor for use in the treatment or prevention of endometriosis and/or symptoms associated therewith are envisaged.

## Description

### FIELD OF THE INVENTION

The present invention relates to methods and compounds for the treatment and prevention of endometriosis or symptoms associated therewith in a patient.

### BACKGROUND OF THE INVENTION

Endometriosis is a common gynaecological condition in which cells from the lining of the uterus (endometrium) appear and flourish outside the uterine cavity, most commonly on the pelvic peritoneum, the ovaries and/or the rectovaginal septum, and more rarely in the pericardium, pleura, or even in the brain. It has been estimated that the prevalence of pelvic endometriosis approaches 6-10% in the general female population in the reproductive age. Endometriosis is a benign disorder which, in a sub-population of patients, may develop into an aggressive disease. Endometriosis is associated with various distressing symptoms including dysmenorrhoea, dyspareunia, pelvic pain, infertility and menstrual irregularities.

Endometriosis may be treated medically or surgically, or via a combination thereof. The patient's symptoms and desire for childbearing dictate appropriate therapy. Not all therapy works for all patients. In most cases, treatment gives patients significant relief from pelvic pain and assists them in achieving pregnancy.

Surgical treatment typically involves removal of the ectopic lesion tissue, and can be either conservative, aiming to preserve the reproductive potential of the patient, or comparatively radical for severe disease, involving dissection of the urinary tract, bowel, and rectovaginal septum, or total abdominal hysterectomy and bilateral salpingo- oopherectomy.

Medical pharmacological endometriosis treatments known in the art include androgenic therapies (e.g. by administration of hormones such as danazol or gestrinone), the administration of GnRH agonists (e.g. buserelin, goserelin, leuprolide, nafarelin and triptorelin) or GnRH antagonists (e.g. cetrorelix and abarelix), and the administration of progestogens such as medroxyprogesterone acetate.

However, these approaches are not without unwanted side effects. For example, danazol and gestrinone may cause weight gain, hirsuitism, acne, mood changes and metabolic effects on the cardiovascular system. The group of GnRH agonists and antagonists are found to cause a profound suppression of estrogen to post-menopausal levels leading to depletion of bone mineral density, which restricts their use to only six months of therapy.

Progestogens cause side effects such as irregular bleeding, bloating, weight gain and metabolic effects on the cardiovascular system.

Moreover, the underlying process that causes endometriosis may not cease after surgical or medical intervention. Studies have shown that endometriosis recurs at a rate of 20 to 40 percent within five years following conservative surgery, unless hysterectomy is performed or menopause reached.

Thus, there is still need for methods for treatment of endometriosis and/or the treatment and prevention of one or more symptoms and disorders associated therewith, without significant adverse effects or the limited efficacy of currently available therapies.

Macrophage Activating Factors (MAFs) are Iymphokines that prime macrophages to become cytotoxic to tumors. These factors also control the expression of la antigens on the macrophage cell surface. European patent application EP0837932 provides indications that the use of MAFs, particularly the GcMAF protein, is effective in the treatment of certain cancers and HIV. However, the use of MAFs for the treatment and prevention of endometriosis has not been suggested.

### SUMMARY OF THE INVENTION

The present invention relates to methods and compounds for the treatment of endometriosis in a patient and/or the treatment and prevention of one or more symptoms and disorders associated with endometriosis.

In a first aspect, the present invention provides a Macrophage Activating Factor (MAF), more particularly a composition comprising at least a Macrophage Activating Factor (MAF), for use in the treatment of endometriosis in a (female) subject and for the treatment and prevention of symptoms thereof. Indeed, the present inventors have found that administration of a MAF, particularly GcMAF, leads to a significant decrease of the manifestation of the symptoms of endometriosis, without causing significant side effects.

In particular embodiments of the compositions of the invention, the Macrophage Activating Factor is GcMAF.

In particular embodiments, the composition further comprises one or more other active ingredients. In further embodiments, one of said one or more other active ingredients is Vitamin B12. In other embodiments, the Macrophage Activating Factor is the only active ingredient.

In certain embodiments, the composition is used for reducing one or more signs or symptoms of endometriosis selected from the group consisting of pelvic pain, dysmenorrhoea, dyspareunia, dysuria, irregular menstruation, amenorrhoea, pre-menstrual syndrome, abdominal pain, fatigue, infertility, and constipation. In particular embodiments, the composition is used for reducing dysmenorrhoea and abdominal pain, more particularly in patients suffering from endometriosis.

In particular embodiments, the subject is also diagnosed with Chronic Fatigue Syndrome (CFS).

In certain embodiments, the invention provides for the use of the compositions as described above, in a pregnant subject.

The present invention provides methods for the treatment or prevention of endometriosis and/or one or more symptoms associated with endometriosis in a subject, comprising a step of administration to said subject of a Macrophage Activating Factor.

### DETAILED DESCRIPTION OF THE INVENTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" when referring to recited components, elements or method steps also include embodiments which "consist of" said recited components, elements or method steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The values as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that each value as used herein is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The present invention relates to the use of Macrophage Activating Factors ("MAFs"), particularly GcMAF, in the treatment or prevention of endometriosis in a subject. The term "Macrophage activating factor" or "MAF" as referred to herein designates a Iymphokine protein that stimulates monocytes and/or macrophages (designated as macrophages hereafter). This stimulation results in the macrophages to become cytotoxic to tumors, and/or primes the phagocytosis of pathogens by macrophages.

The term "GcMAF" as referred to herein designates a Gc (group specific component) protein derived macrophage activating protein, or a domain III (natural or cloned) derived macrophage activating protein (e.g. US patent 6410269, Yamamoto, which is included herein by reference). The term "domain 111" as referred to herein designates the domain III region of the Gc protein. The term "Gc protein" as referred to herein designates a Vitamin D binding protein (DBP); DBP is also known as gc-globulin.

The term "endometriosis" as used herein refers to a condition in which tissue more or less perfectly resembling the uterine mucous membrane (the endometrium) and containing typical endometrial glandular and stromal elements occurs aberrantly in various locations preferentially in the pelvic cavity (ectopic endometrium) and generally is to be found in the region of the ovary, peritoneum, or recto-vaginal wall. However, it should be noted that ectopic endometrium tissue has been found in locations as disparate as the brain and lungs. Endometrial tissue may be determined histologically by looking for endometrial glands and stromal elements or by using markers.

The term "treatment of endometriosis" as used herein includes treatment to reduce (or remove) the amount of endometrial tissue which is present outside the uterine cavity and/or treatment to reduce and/or ameliorate one or more signs or symptoms associated with endometriosis.

In a first aspect, the present invention provides a composition comprising a Macrophage Activating Factor (MAF) for use in the treatment or prevention of endometriosis in a (female) subject, typically a mammal such as a human. In particular embodiments, the present invention provides for a treatment that reduces, inhibits or decreases one or more of the symptoms of endometriosis.

Studies have suggested that immunologic factors may play a role in the pathogenesis of endometriosis and endometriosis-associated infertility. However, the use of MAFs for the prevention and treatment of endometriosis has not been suggested. The present inventors surprisingly found that use of MAF in patients suffering from endometriosis leads to significant reduction of at least some of the symptoms of endometriosis, without significant side effects. Moreover, in contrast with classic treatments of endometriosis which attempt to mimic either menopause or pregnancy, treatment with the compositions of the present invention does not interfere with the normal menstrual cycle.

In particular embodiments, the MAF comprised by the composition described herein is GcMAF or a derivative thereof mimicking the activity of GcMAF. Such derivatives have been described in the art, such as the protein described by Yamamoto et al. (Cancer Res 57: 295-299, 1997) consisting of Gc protein domain III (macrophage activating site) that has 85 amino acids from the C-terminal (458) to the 85th amino acid (374) having the GaINAc moiety and the peptides described by Schneider et al. (35) (Crit Rev Eukaryot Gene Expr 13: 277-284, 2003). In particular embodiments, the composition comprises a protein comprising a sequence of at least 14 amino acids identical to GcMAF or a sequence of at least 20 amino acids having at least 70% sequence identity to GcMAF. The inventors have found that compositions comprising GcMAF or GcMAF like activity are particularly effective for the treatment or prevention of endometriosis.

It has been found that administration of the compositions described herein to a patient diagnosed with endometriosis may result in mitigation of one or more signs and/or symptoms of endometriosis. Accordingly, in particular embodiments, the composition comprising a MAF as described herein is used for the treatment of one or more signs and/or symptoms of endometriosis. In further embodiments, these signs and/or symptoms are selected from the group consisting of (chronic) pelvic pain, dysmenorrhea, dyspareunia, dysuria, irregular menstruation, amenorrhoea, pre-menstrual syndrome, abdominal pain, (chronic) fatigue, infertility, and constipation. In further particular embodiments, the invention relates to the use of the compositions described herein for the treatment of amenorrhoea and/ abdominal pain (such as dysmenorrheal), more particularly in patients with endometriosis.

Indeed it is envisaged that the compounds of the invention can be used for the treatment and prevention of one or more symptoms associated with endometriosis, even if the actual diagnosis of endometriosis has not been made. More particularly, the present invention envisages the use of the compositions described herein for the treatment of a patient having at least two, more particularly at least three, most particularly at least four of the symptoms selected from, abdominal and/or pelvic pain (including dysmenorrhea), dyspareunia, dysuria, irregular menstruation or amenorrhoea, pre-menstrual syndrome, (chronic) fatigue, infertility, and constipation. In further particular embodiments, the invention relates to the use of the compositions described herein for the treatment of amenorrhoea and/ abdominal pain (such as dysmenorrheal), more particularly in patients with endometriosis. More particularly, the compositions are envisaged for the treatment of patients characterized by irregular menstruation and/ abdominal pain (such as dysmenorrhea). In particular embodiments, the symptoms are not attributable to a cancer such as endometrial cancer.

In particular embodiments, the present invention provides for compositions comprising a MAF, which are capable of reducing one or more of the signs or symptoms of endometriosis by at least 10%, more particularly at least 20%, 30%, 40%, 50%, 60%, 70% or 80% or more, compared to prior to administration of the composition of the invention.

It has further been found that patients suffering from endometriosis and/or one or more symptoms associated therewith are characterized by an increased level of serum nagalase. More particularly, the compositions are envisaged for the treatment of a subject suffering from endometriosis having an increased serum nagalase (alpha-N-acetylgalactosaminidase) activity compared to a healthy control. Healthy individuals are typically characterized by a serum nagalase activity which is lower than 0.9 nmol.mg⁻¹.min⁻¹, more preferably lower than 0.9 nmol.mg⁻¹.min⁻¹,. Accordingly, in certain embodiments, the subject to be treated has (more particularly is established to have) a serum nagalase activity higher than 0.9 nmol.mg⁻¹.min⁻¹. In further embodiments, the serum nagalase activity is higher than 1 nmol.mg-¹.min⁻¹, 1.25 nmol.mg⁻¹.min⁻¹, or 1.5 nmol.mg⁻¹.min⁻¹. A method for determining serum nagalase activity has been described by Korbelik et al. (British Journal of Cancer 1998, vol. 77, page 1009-1014, hereby incorporated by reference). In further particular embodiments, the patient envisaged for treatment has been diagnosed with endometriosis and an increased serum nagalase activity.

Studies have indicated that women with endometriosis have a higher risk of premature birth or other pregnancy complications, as well as being more likely to give birth through Caesarean section. The compositions comprising one or more MAFs described herein, particularly GcMAF may be administered during pregnancy. Accordingly, in particular embodiments, the subject is pregnant.

In particular embodiments, the invention envisages the use of the compositions of the present invention for the treatment of patients suffering from both endometriosis and another disease, such as Chronic Fatigue Syndrome (CFS), also known as Myalgic Encephalomyelitis (ME). Whereas compounds suitable for treatment against endometriosis typically are not suitable for treatment of CFS and vice versa, the inventors have found that administration of GcMAF is also effective for treating chronic fatigue. Accordingly, in particular embodiments, the present invention relates to methods for treatment of a patient suffering from endometriosis and Chronic Fatigue Syndrome (CFS). The term "chronic fatigue syndrome" or "CFS" as referred to herein designates a condition which is diagnosed based on the following criteria (as developed by the U.S. Centers for Disease Control and Prevention in 1994):
1. Clinically evaluated, unexplained persistent or relapsing chronic fatigue that is of new or definite onset (i.e., not lifelong), is not the result of ongoing exertion, is not substantially alleviated by rest, and results in substantial reduction in previous levels of occupational, educational, social, or personal activities.
2. The concurrent occurrence of four or more of the following symptoms: substantial impairment in short-term memory or concentration; sore throat; tender lymph nodes; muscle pain; multi-joint pain without swelling or redness; headaches of a new type, pattern, or severity; unrefreshing sleep; and post-exertional malaise lasting more than 24 hours. These symptoms must have persisted or recurred during 6 or more consecutive months of illness and must not have predated the fatigue.

Several standardized tests exist to follow up patients suffering from CFS/ME, such as the Activities of Daily Living Questionnaire (ADL)(see e.g. Collin et al. Int. Disabil. Stud. 10:61-63, 1988), the (Cognitive Deficit Subset of the) Symptom Checklist Questionnaire (SCL-90-R) (see e.g. Diaz-Mitoma et al. Journal of Chronic Fatigue Syndrome 11:71-95, 2003) and the Karnofsky Performance Score (KPS) (Karnofsky D and Burchenal JH. Clinical evaluation of chemotherapeutic agents in cancer. In Macleod CM. (ed) Evaluation of chemotherapeutic agents. Columbia University Press, New York 1949; 199-205). An alternative performance score specifically developed for CFS/ME patients is the Bell disability scale (D.S. Bell, The Doctor's Guide to Chronic Fatigue Syndrome, Reading, MA: Addison-Wesley, 1994).

Further indications of CFS/ME also include an increased amount of metabolites in physiological fluids such as urine. The metabolite concentration in physiological fluids can be detected and monitored using reducible dyes, which allows monitoring of the disease (W02010142322, which is hereby incorporated by reference). In particular embodiments, the subject is diagnosed with aberrant and/or increased metabolite production.

In particular embodiments the compositions of the present invention are envisaged to reduce or prevent one or more symptoms selected from of substantial impairment in short-term memory or concentration, sore throat, tender lymph nodes, muscle pain, multi-joint pain without swelling or redness, headaches of a new type, pattern, or severity; unrefreshing sleep, post-exertional malaise lasting more than 24 hours, widespread pain, fatigue, feeling run down, sluggish muscle cramps and pains, unexplained or excessive weight gain, inability to lose weight, gastrointestinal problems, irritable bowel syndrome, poor sleeping, headaches and migraines, constipation, orthostatism and exhaustion and/or one or more symptoms selected from the group consisting of (chronic) pelvic pain, dysmenorrhea, dyspareunia, dysuria, irregular menstruation, amenorrhoea, pre-menstrual syndrome, abdominal pain, infertility. In particular embodiments, the compositions are envisaged for use in the treatment or prevention of fatigue, amenorrhoea and abdominal pain. More particularly the compositions are envisaged for the treatment of one or more of the above-recited symptoms in a patient suffering from CFS and endometriosis.

The present invention relates to a MAF for use in the treatment or prevention of endometriosis in a subject. In particular embodiments, the present invention relates to use of a MAF for the manufacture of a medicament for the treatment or prevention of endometriosis in a subject. In particular embodiments, the present invention relates to a MAF for use in the treatment or prevention of endometriosis in a subject.

In certain embodiments, the present invention provides in compositions as described above, comprising a pharmaceutically acceptable excipient and a therapeutically effective amount of said MAF. In further embodiments, the MAF is GcMAF.

In particular embodiments, the compositions of the present invention comprise MAF as the sole active ingredient or consist of a MAF. However, treatment regimes are also envisaged wherein the MAF is administered in combination with one or more other therapeutic compounds.

In particular embodiments, the present invention relates to the therapeutic use of a MAF as described above used in combination with other medical or surgical treatments for endometriosis, for example hormonal treatments, GnRH agonists, GnRH antagonists, etc. in certain embodiments, surgical treatment or medical treatment may be used prior, during or after treatment with the composition described herein.

The inventors further discovered positive synergistic effects when the MAF of the present invention was used in combination with Vitamin B12. In particular embodiments, the present invention thus relates to the therapeutic use of a MAF as described above used in combination with Vitamin B12. In particular embodiments, the MAF of the present invention and Vitamin B12 are administered to the patient separately.

The inventors also discovered positive synergistic effects when the MAF of the present invention was used in combination with one or more compounds selected from interferon alpha, interferon beta, interferon gamma and/or interleukin-7. In particular embodiments, the present invention relates to the therapeutic use of a MAF according to the present invention used in combination with one or more compounds selected from interferon alpha, interferon beta, interferon gamma and/or interleukin-7. In particular embodiments, the MAF of the present invention and interferon alpha, interferon beta, interferon gamma and/or interleukin-7 are administered to the patient separately.

As described hereabove, in the treatment regimens envisaged in the context of the present invention the MAF can be administered separately or simultaneously with another compound of interest. When administered simultaneously, the compounds may be comprised in the same or separate formulations. However, in particular embodiments, the invention provides compositions for combined treatment regimens. Thus, in particular embodiments, the present invention relates to a MAF or composition as described above, and/or to the use thereof, wherein said MAF is provided in combination with at least one other pharmacologically active compound, for example, vitamin B12, interferon alpha, interferon beta, interferon gamma and/or interleukin-7. In particular embodiments the composition comprises, or further comprises Vitamin B12. In particular embodiments, the composition comprises, or further comprises one or more compounds selected from interferon alpha, interferon beta, interferon gamma and/or interleukin-7.

The present invention provides a use of a MAF or composition as described above in the manufacture of a medicament for the treatment or prevention of endometriosis in a subject, wherein said MAF is provided in a conjugate or in a pharmaceutical composition further comprising a pharmaceutically acceptable excipient and a therapeutically effective amount of said MAF.

The term "therapeutically effective amount" as used herein means that amount of MAF, compound, conjugate or pharmaceutical agent that elicits the biological or medicinal response in a tissue, system, animal or human that is being sought by a researcher, veterinarian, medical doctor or other clinician, which includes alleviation of the symptoms of the disease being treated.

The pharmaceutical composition can be prepared in a manner known per se to one of skill in the art. For this purpose, at least one MAF, one or more solid or liquid pharmaceutical excipients and, if desired, in combination with at least one other pharmaceutical active compound, are brought into a suitable administration form or dosage form which can then be used as a pharmaceutical in human medicine or veterinary medicine.

Particular forms of the pharmaceutical composition may be, for example, solutions, suspensions, emulsions, creams, tablets, pills, capsules, nasal sprays, liposomes or micro-reservoirs, especially compositions in orally ingestible or sterile injectable form, for example, as sterile injectable aqueous or oleaginous suspensions or suppositories and sterile packaged powders (which are usually reconstituted prior to use) for administration as a bolus and/or for continuous administration. The solid carrier may comprise one or more excipients, e.g. lactose, dextrose, sucrose, sorbitol, mannitol, starches, gum acacia, calcium phosphate, alginates, tragacanth, gelatin, calcium silicate, microcrystalline cellulose, polyvinylpyrrolidone, polyethylene glycol, fillers, disintegrating agents, binders, e.g. cellulose, carboxymethylcellulose or starch or anti-stick agents, e.g. magnesium stearate, to prevent tablets from adhering to tabletting equipment, (sterile) water, methylcellulose, methyl- and propylhydroxybenzoates, talc, magnesium stearate, edible oils, vegetable oils and mineral oils or suitable mixtures thereof. Tablets, pills and boluses may be formed so as to disintegrate rapidly or to provide slow release of the active ingredient. The formulations can optionally contain other pharmaceutically active substances (which may or may not lead to a synergistic effect with the MAF of the invention) and other substances that are commonly used in pharmaceutical formulations, such as lubricating agents, wetting agents, emulsifying, and suspending agents, dispersing agents, desintegrants, bulking agents, fillers, preserving agents, sweetening agents, flavoring agents, flow regulators, release agents, etc. The compositions may also be formulated so as to provide rapid, sustained, or delayed release of the active compound(s) contained therein, for example using liposomes or hydrophilic polymeric matrices based on natural gels or synthetic polymers.

For the purposes of the present invention, the MAF or compounds or the pharmaceutical composition of the present invention may be administered by any of several routes including but not limited to oral administration, buccal (e.g. sub-lingual) administration, topical administration and parenterally injection, i.e. including intravenous, intraperitoneal, intramuscular, intrasternal or subcutaneous injection. The MAF or compound of the present invention will generally be administered in an "effective amount", by which is meant any amount of a MAF that, upon suitable administration, is sufficient to achieve the desired therapeutic or prophylactic effect in the individual to which it is administered. Usually, depending on the patient and the route of administration, such an effective amount will usually be between 0.01 to 100 ng, preferably between 0.1 and 50 ng and even more preferably between 0.1 and 10 ng of the MAF per kilogram body weight, which may be administered as a weekly dose, a single daily dose, divided over one or more daily doses, or essentially continuously, e.g. using a drip infusion.

In certain embodiments, the effective amount is between 25 and 500 ng GcMAF/dose. In further particular embodiments, the effective amount is between 25-200ng GcMAF/dose, such as 100 ng/dose.

In particular embodiments, the MAF or pharmaceutical composition of the present invention are administered once per week, during 2 to 50 weeks, preferably during 5 to 40 weeks, such as during 12 weeks.

The amount(s) to be administered, the route of administration and the further treatment regimen may be determined by the treating clinician, depending on factors such as the age, gender and general condition of the patient and the nature and severity of the symptoms to be treated.

In accordance with the method of the present invention, said pharmaceutical composition can be administered separately at different times during the course of therapy or concurrently in divided or single combination forms. The present invention is therefore to be understood as embracing all such regimes of simultaneous or alternating treatment and the term "administering" is to be interpreted accordingly.

For an oral administration form, the compositions of the present invention can be mixed with suitable additives, such as excipients, stabilizers or inert diluents, and brought by means of the customary methods into the suitable administration forms, such as tablets, coated tablets, hard capsules, aqueous, alcoholic, or oily solutions. Examples of suitable inert carriers are gum arabic, magnesia, magnesium carbonate, potassium phosphate, lactose, glucose or starch, in particular, corn starch. In this case, the preparation can be carried out both as dry and as moist granules. Suitable oily excipients or solvents are vegetable or animal oils, such as sunflower oil or cod liver oil. Suitable solvents for aqueous or alcoholic solutions are water, glycerol and sugar solutions. Polyethylene glycols and polypropylene glycols are also useful as further auxiliaries for other administration forms. As immediate release tablets, these compositions may contain microcrystalline cellulose, dicalcium phosphate, starch, magnesium stearate and lactose and/or other excipients, binders, extenders, disintegrants, diluents and lubricants known in the art.

The oral administration of a pharmaceutical composition comprising the MAF according to the invention, is suitably accomplished by uniformly and intimately blending together a suitable amount of said MAF in the form of a powder, optionally also including a finely divided solid carrier, and encapsulating the blend in, for example, a hard gelatin capsule. The solid carrier can include one or more substances, which act as binders, lubricants, disintegrating agents, coloring agents, and the like. Suitable solid carriers include, for example, calcium phosphate, magnesium stearate, talc, sugars, lactose, dextrin, starch, gelatin, cellulose, polyvinylpyrrolidine, low melting waxes and ion exchange resins.

Oral administration of a pharmaceutical composition comprising the MAF according to the present invention can also be accomplished by preparing capsules or tablets containing the desired amount of said MAF, optionally blended with a solid carrier as described above. Compressed tablets containing the pharmaceutical composition of the invention can be prepared by uniformly and intimately mixing the active ingredient with a solid carrier such as described above to provide a mixture having the necessary compression properties, and then compressing the mixture in a suitable machine to the shape and size desired. Molded tablets maybe made by molding in a suitable machine, a mixture of powdered compound or MAF according to the invention moistened with an inert liquid diluent.

For parenterally administration such as subcutaneous or intravenous administration, the MAF or compounds of the present invention, if desired with the substances customary therefore such as solubilizers, emulsifiers or further auxiliaries, are brought into solution, suspension, or emulsion. The MAF or compounds of the invention can also be lyophilized and the Iyophilizates obtained used, for example, for the production of injection or infusion preparations. Suitable solvents are, for example, water, physiological saline solution or alcohols, e.g. glycerol, in addition also sugar solutions such as glucose or mannitol solutions, or alternatively mixtures of the various solvents mentioned. The injectable solutions or suspensions may be formulated according to known art, using suitable nontoxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

For topical administration, which includes the application of medicinal substances to the skin or various body orifices, the MAF or compounds of the present invention may be applied in a variety of forms such as liquid, semisolid or solid. Some of the possible components of topical, transdermal and transmucosal formulations and delivery devices include solubilizing agents, suspending agents, dispersing agents, preservatives, animal and vegetable fats, oils, or waxes, emollients, stabilizing agents, thickening or gelling agents, buffering agents, adhesive agents, adjuvants and additives, emulsifiers and penetration enhancing agents as known to the person skilled in the art.

For sub-lingual administration, the MAF or compounds of the present invention may be formulated in admixture with at least a proper excipient typical of sublingual formulations. As non limiting example for the artisan skilled in the art, said excipients are selected from the group including: water-soluble inert excipients (such as, for example, mannitol, sorbitol, lactose); water-insoluble excipients which help the delivery of the active substance at the sublingual mucosa level (such as, for example, microcrystalline cellulose); sweeteners (such as, for example, aspartame, sodium saccharate); flavourings (such as, for example, peach, apricot, banana, strawberry, orange, mandarin flavours); lubricants (such as, for example, magnesium stearate, PEG 6000); taste correctors (such as, for example, sodium citrate); other excipients, additives, carriers commonly used in the formulation pharmaceutical art.

In addition to administration with conventional carriers, the MAF or compounds of the present invention may be administered by a variety of specialized oligonucleotide or nucleic acid delivery techniques such as by encapsulation in various encapsulating materials, such as in unilamellar liposomes (Bayard et al., Eur. J. Biochem. 151: 319, 1985) or by conjugation to carrier molecules such as poly(L-lysine). Reconstituted Sendai virus envelopes have been successfully used to deliver RNA and DNA to cells (Arad et al., Biochem. Biophys. Acta. 85: 88, 1986). Moreover, the virus envelope is not limited to Sendai virus, but could include encapsulation in any retroviral amphotrophic particle. These techniques may be utilized for introduction of the present Macrophage Activation Factors into cells.

In a further aspect, the present invention provides a method for the treatment or prevention of endometriosis in a subject. The present method comprises a step of administration to said subject of a MAF, particularly GcMAF. In certain embodiments, the method comprises a step of administration to the subject of a composition as described herein.

In a further aspect, the present invention provides the use of a MAF for the treatment or prevention of endometriosis in a subject. In particular embodiments, the MAF is GcMAF. In certain embodiments, the present invention provides the use of a composition comprising a MAF as described herein for the treatment or prevention of endometriosis in a subject. In certain embodiments, the present invention provides the use of a MAF in a method for the treatment or prevention of endometriosis as described above.

The following examples are provided for the purpose of illustrating the present invention and by no means are meant and in no way should be interpreted to limit the scope of the present invention.

### Example

Three adult patients were diagnosed with endometriosis via laparoscopy. The patients suffered from various signs and symptoms such as amenorrhoea or irregular menstruation, pre-menstrual syndrome, and abdominal pains. The patients were treated by weekly subcutaneous administration of 50-100 ng GC-MAF, during 12 weeks. After treatment, most signs and symptoms were significantly reduced or had disappeared. The menstrual cycle of all patients was restored or normalized.

It was determined that the serum of these patients suffering from endometriosis prior to treatment showed high serum nagalase activity. After treatment, the serum nagalase activity had decreased to healthy levels.

## Claims

1. A composition comprising a Macrophage Activating Factor for use in the treatment of endometriosis in a subject.

2. The composition according to claim 1, wherein said Macrophage Activating Factor is GcMAF.

3. The composition according to claim 1 or 2, for reducing one or more signs or symptoms of endometriosis selected from the group consisting of pelvic pain, dysmenorrhoea, dyspareunia, dysuria, irregular menstruation, amenorrhoea, pre-menstrual syndrome, abdominal pain, fatigue, infertility, and constipation.

4. The composition according to any one of claims 1 to 3, in which said Macrophage Activating Factor is the only active ingredient.

5. The composition according to any one of claims 1 to 3, further comprising one or more other active ingredients.

6. The composition according to claim 5, where one of said one or more other active ingredients is Vitamin B12.

7. The composition according to any one of claims 1 to 6, wherein said subject is pregnant.

8. The composition according to any one of claims 1 to 7, which is a composition for intravenous delivery.

9. A method for the treatment of endometriosis in a subject, comprising a step of administration to said subject of a composition comprising a Macrophage Activating Factor.

10. The method of claim 9, wherein said patient is treated by intravenous administration of said composition.

11. A composition comprising a Macrophage Activating Factor for use in the treatment and prevention of amenorrhea and abdominal pain in a patient suffering from said symptoms which has been diagnosed to display an increased nagalase serum activity.

12. A method for determining whether or not a patient suffering from amenorrhea and abdominal pain is susceptible to the treatment with a composition comprising a Macrophage Activating Factor, which method comprises, determining the level of serum nagalase activity in a sample of said patient, wherein an increased level of serum nagalase activity is indicative of the susceptibility of said treatment.
